(19) Europäisches Patentamt
European Patent Office
Office européen des brevets

(11) Veröffentlichungsnummer: **0 121 081**
**B1**

# (12) EUROPÄISCHE PATENTSCHRIFT

(45) Veröffentlichungstag der Patentschrift: **30.01.91**

(21) Anmeldenummer: **84101905.2**

(22) Anmeldetag: **23.02.84**

(51) Int. Cl.[5]: **C 07 D 249/08,** C 07 D 233/58, C 07 D 233/60, C 07 D 405/08, C 07 D 401/08, C 07 D 409/08, A 61 K 31/41, A 61 K 31/415, A 01 N 43/64, A 01 N 43/50

## (54) Azolylmethylcycloalkane, Verfahren zu ihrer Herstellung und ihre Verwendung als Arzneimittel.

(30) Priorität: **03.03.83 DE 3307477**
**03.03.83 DE 3307479**

(43) Veröffentlichungstag der Anmeldung:
**10.10.84 Patentblatt 84/41**

(45) Bekanntmachung des Hinweises auf die Patenterteilung:
**30.01.91 Patentblatt 91/05**

(84) Benannte Vertragsstaaten:
**AT BE CH DE FR GB IT LI NL SE**

(56) Entgegenhaltungen:
**EP-A-0 000 950**
**EP-A-0 000 951**
**EP-A-0 003 560**
**EP-A-0 063 099**
**EP-A-0 064 245**
**DE-A-2 735 872**
**DE-A-2 855 329**
**US-A-3 575 999**
**US-A-3 927 017**
**US-A-4 160 838**
**US-A-4 160 841**

(73) Patentinhaber: **BASF Aktiengesellschaft**
**Carl-Bosch-Strasse 38**
**D-6700 Ludwigshafen (DE)**

(72) Erfinder: **Janssen, Bernd, Dr.**
**Leuschnerstrasse 18 a**
**D-6700 Ludwigshafen (DE)**
Erfinder: **Meyer, Norbert, Dr.**
**Stahlbuehlring 155 A**
**D-6802 Ladenburg (DE)**
Erfinder: **Pommer, Ernst-Heinrich, Dr.**
**Berliner Platz 7**
**D-6703 Limburgerhof (DE)**
Erfinder: **Ammermann, Eberhard, Dr.**
**Sachsenstrasse 3**
**D-6700 Ludwigshafen (DE)**
Erfinder: **Kohlmann, Friedrich-Wilhelm, Dr.**
**An der Duene 6**
**D-2082 Morrege (DE)**
Erfinder: **Wesenberg, Walter**
**Dorfstrasse 16**
**D-2421 Bujendorf ueber Eutin (DE)**
Erfinder: **Heberle, Wolfgang, Dr.**
**Gebelsbergstrasse 41**
**D-7000 Stuttgart (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 121 081 B1

**Beschreibung**

Die vorliegende Erfindung betrifft neue Azolylmethylcycloalkane, Verfahren zu ihrer Herstellung und diese enthaltende Mittel, die als Antimykotika und Fungizide verwendet werden können, sowie deren Verwendung bei der Heilung von Krankheiten.

Es sind bereits zahlreiche antimykotische Mittel, z.B. Azolylmethylcarbinole wie Miconazol (DE—OS 19 40 388) oder auch Azolylmethyldioxolane wie Ketoconazol (DE—OS 28 04 096), bekannt. Ihre Wirkungen befriedigen nicht immer (Lit.: Chemotherapy *22*, 1 (1976); Dtsch. Apoth. Ztg. *118*, 1269 (1978); Z. Hautkr. *56*, 1109 (1981); Am. Rev. Respir. Dis. *126*, 171 (1982); Selecta *49*, 4602 (1982). Weiter ist bekannt, Azolverbindungen wie Azolylmethylcarbinole oder Azolylmethylketone (DE—OS 24 31 407, FR—PS 22 49 616) als Fungizide zu verwenden. Ihre Wirkung ist jedoch unbefriedigend. Es sind ferner Cycloalkylmethylazole bekannt, von denen einige eine fungizide Wirkung haben (DE—A—2 855 329, EP—A—0 000 951, US—A—3 927 017, EP—A—0 063 099, DE—A—2 735 872, US—A—3 575 999, US—A—4 160 838, US—A—4 160 841).

Der vorliegenden Erfindung liegt die Aufgabe zugrunde, neue Verbindungen zur Verfügung zu stellen, die eine bessere antimykotische und fungizide Wirkung besitzen.

Es wurde nun gefunden, daß Azolylmethylcycloalkane der Formel I

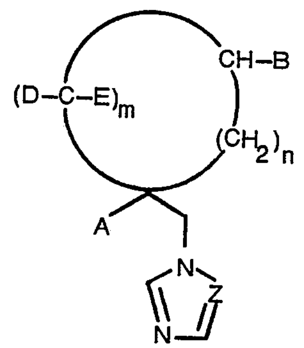

(I),

in der

A und B gleich oder verschieden sind und Wasserstoff, Alkyl mit 1 bis 4 C-Atomen, die ggf. durch Halogen substituiert sein können, Naphthyl, Furanyl, Thienyl, Pyridyl, Biphenyl oder Phenyl bedeuten, wobei der Phenylrest durch Halogen, Nitro, Alkyl, Alkoxy oder Halogenalkyl mit jeweils 1 bis 4 C-Atomen, Phenoxy oder den Phenylsulfenylrest substituiert sein kann,

D und E gleich oder verschieden sind und Wasserstoff oder Halogen bedeuten,

Z CH oder N bedeutet,

m 1 und

n 0 bedeuten,

sowie deren verträgliche Säureadditionssalze und Metallkomplexe gute antimikrobielle, insbesondere antimykotische und fungizide Eigenschaften aufweisen.

Die neuen Verbindungen der Formel I enthalten chirale Zentren und werden im allgemeinen in Form von Racematen erhalten. Während sich die neuen Azolylmethylcyclopropane der Formel I ($m = 1, n = 0$; A, B ungleich H) einfach diasteroselektiv synthetisieren lassen, erhält man im allgemeinen Gemische aus erythro- und threo-Formen. In diesem Fall lassen sich die Diastereomeren bei den neuen Verbindungen beispielsweise durch Löslichkeitsunterschiede oder durch Säulenchromatographie trennen un din reiner Form isolieren. Aus den einheitlichen Diastereomerenpaaren kann man nach bekannten Methoden einheitliche Enantiomere erhalten. Sowohl diese als auch ihre Gemische (Racemate) werden von der vorliegenden Erfindung umfaßt. Zur Herstellung antimikrobieller bzw. fungizide Mittel kann man sowohl die einheitlichen Diastereomeren bzw. Enantiomeren wie auch deren Gemische verwenden.

A und B bedeuten beispielsweise Wasserstoff, Methyl, Ethyl, Propyl, Isopropyl, Butyl, sek. Butyl, iso-Butyl, tert.-Butyl, Trifluormethyl, 1-Naphthyl, 2-Naphthyl, p-Biphenyl, Phenyl, 2-Chlorphenyl, 3-Chlorphenyl, 4-Chlorphenyl, 4-Fluorphenyl, 4-Bromphenyl, 2,4-Dichlorphenyl, 3,4-Dichlorphenyl, 3,5-Dichlorphenyl, 2,6-Dichlorphenyl, 3-Chlor-4-methylphenyl, 2-Methoxyphenyl, 3-Methoxyphenyl, 2,4-Dimethoxyphenyl, 3,4-Dimethoxyphenyl, 4-Methoxyphenyl, 4-Ethoxyphenyl, 4-tert.Butoxyphenyl, 4-Methylphenyl, 4-Ethylphenyl, 4-Isopropylphenyl, 4-tert.Butylphenyl, 4-Phenoxyphenyl, 3-Phenoxyphenyl, 3-Nitrophenyl, 4-Nitrophenyl, 3-Trifluormethylphenyl, 4-Trifluormethylphenyl, 4-Phenylsulfenylphenyl, 3-Furanyl, 2-Furanyl, 3-Thienyl, 2-Thienyl, 2-Pyridyl, 3-Pyridyl, 4-Pyridyl.

D und E bedeuten beispielsweise Fluor, Chlor, Brom und vorzugsweise Wasserstoff.

Bei der Anwendung der neuen Substanzen als Antimykotika kommen als Säuren zur Bildung physiologisch verträglicher Salze vorzugsweise Halogenwasserstoffsäuren, wie z.B. Bromwasserstoffsäure und insbesondere Chlorwasserstoffsäure, mit der die erfindungsgemäßen Verbindungen besonders gut kristallisierende Salze bilden, in Frage. Ferner sind zu nennen: Phosphorsäure, Salpetersäure,

Schwefelsäure, mono- und bifunktionelle Carbonsäuren und Hydroxycarbonsäuren — wie Essigsäure, Oxalsäure, Maleinsäure, Bernsteinsäure, Fumarsäure, Weinsäure, Zitronensäure, Salicylsäure, Sorbinsäure, Milchsäure — sowie Sulfonsäuren — wie p-Toluolsulfonsäure und 1,5-Naphthalindisulfonsäure —.

Für die Anwendung als Fungizide kommen als Säureadditionssalze beispielsweise die Hydrochloride, Bromide, Sulfate, Nitrate, Phosphate, Oxalate oder Dodecyl-benzolsulfonate in Betracht. Die Wirksamkeit der Salze geht auf das Kation zurück, so daß Wahl des Anions an sich beliebig ist. Aus praktischen Gründen werden jedoch nichtphytotoxische Anionen verwendet.

Die Salze werden hergestellt durch Umsetzung der Azolylmethylcycloalkane mit den entsprechenden Säuren.

Als Metallkomplexe können in fungiziden Mitteln solche aus einer Verbindung der Formel I und einem Metallsalz beispielsweise einem Salz von Kupfer, Zink, Zinn, Mangan, Eisen, Kobalt oder Nickel, mit einer anorganischen Säure, z.B. Salzsäure, Schwefelsäure, Phosphorsäure oder Bromwasserstoffsäure verwendet werden. Die Metallkomplexe werden hergestellt durch Umsetzung der Azolylmethylcycloalkane mit den entprechenden Metallsalzen.

Die neuen antimikrobiell wirksamen Verbindungen der Formel I lassen sich herstellen, indem man

a) ein Cycloalkan der Formel II

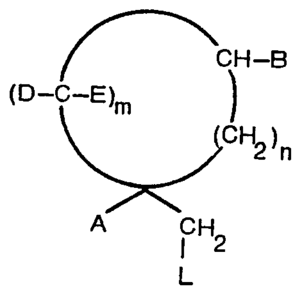

(II),

in der A, B, D, E, m und n die oben angegebene Bedeutung haben und L eine nucleophil substituierbare Abgangsgruppe darstellt, mit einer Verbindung der Formel III

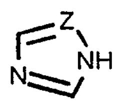

(III),

in der Z die oben angegebene Bedeutung hat, umsetzt oder

b) eine Verbindung der Formel II, in der A, B, D, m und n die oben angegebene Bedeutung haben und L für eine Hydroxygruppe steht, mit einer Verbindung der Formel IV,

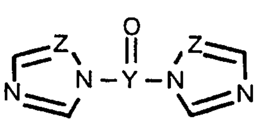

(IV)

in welcher Z die oben angegebene Bedeutung hat, und Y eine Kohlenstoff- oder Schwefelatom bedeutet, in einem aprotischen Lösungsmittel umsetzt

und die so erhaltenen Verbindungen gegebenenfalls in ihre physiologisch verträglichen Säureadditionssalze überführt.

Die Reaktion a) erfolgt vorzugsweise gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C. Zu den bevorzugten Lösungs- und Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Geeignete Basen, die gegebenenfalls auch als säurebindende Mittel bei der Reaktion verwendet werden können, sind beispielsweise Alkalihydroxide wie Lithium-, Natrium- oder Kaliumhydroxid; Alkalicarbonate wie Natrium- oder Kaliumcarbonat oder Natrium- und Kaliumhydrogencarbonat, Überschüsse an 1,2,4-Triazol, Imidazol, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Benzyltriethylammoniumchlorid oder -bromid oder Kronenether wie 12-Krone-4, 15-Krone-5, 18-Krone 6, Dibenzo-18-Krone-6 oder Dicyclohexanon-18-Krone-6 in Frage.

Die Umsetzungen werden im allgemeinen bei Temperaturen zwischen 10 und 120°C drucklos oder unter Druck, kontinuierlich oder diskontinuierlich durchgeführt.

Die Reaktion a) kann auch so durchgeführt werden, daß man die Verbindung III in ein Metallsalz, vorzugsweise ein Alkalisalz, überführt und dieses gegebenenfalls in Gegenwart eines Lösungs- oder Verdünnungsmittels und gegebenenfalls unter Zusatz einer starken anorganischen oder organischen Base und gegebenenfalls unter Zusatz eines Reaktionsbeschleunigers bei Temperaturen zwischen −10 und +120°C mit der Verbindung II umsetzt. Zu den bevorzugten Lösungs- oder Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsulfoxid und schließlich Sulfolan.

Geeignete Basen, die gegebenenfalls auch als šurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butoxid, Lithium-, Natrium- oder Kalium-Triphenylmethyl und Naphthalin-Lithium, -Natrium oder -Kalium.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Benzyltriethylammoniumchlorid oder -bromid oder Kronenether wie 12-Krone-4, 15-Krone-5, 18-Krone 6, Dibenzo-18-Krone-6 oder Dicyclohexanon-18-Krone-6 in Frage.

Für die Reaktion b) kommen als Lösungs- oder Verdünnungsmittel polare organische Lösungsmittel wie Nitrile, z.B. Acetonitril, Sulfoxide, z.B. Dimethylsulfoxid, Formamide, z.B. Dimethylformamid, Ketone, z.B. Aceton, Ether, z.B. Diethylether, Tetrahydrofuran und insbesondere Chlorkohlenwasserstoffe, z.B. Methylenchlorid und Chloroform in Frage.

Man arbeitet im allgemeinen zwischen 0 und 100°C, vorzugsweise bei 20 bis 80°C. Bei Anwesenheit eines Lösungsmittels wird zweckmäßigerweise beim Siedepunkt des jeweiligen Lösungsmittels gerbeitet.

Bei der Durchführung des Verfahrens b) setzt man auf 1 Mol der Verbindung der Formel II (L = OH), etwa 1 Mol Carbonyl-bis-1,2,4-triazol-(1) bzw. -imidazol-(1) oder vorzugsweise etwa 1 Mol Sulfonyl-bis-1,2,4-triazol-(1) bzw. -imidazol-(1) ein oder erzeugt Sulfonyl-bis-1,2,4-triazol-(1) bzw. -imidazol-(1) in situ.

Die so erhaltenen Verbindungen der Formel I werden nach üblichen Methoden isoliert, gegebenenfalls gereinigt und gegebenenfalls mit Säuren zu Salzen bzw. Metallsalzen zu Komplexen umgesetzt.

Die Ausgangsverbindungen der Formel II sind auf folgenden Wegen zugänglich:

Die Verbindungen der Formel II, in denen L eine nucleophil substituierbare Abgangsgruppe darstellt, lassen sich nach prinzipiell bekannten Synthesemethoden aus den Verbindungen der Formel II (L = OH) durch Umsetzung mit Halogenidüberträgern wie Chlorwasserstoff, Bromwassersoff, Thionylchlorid, Thionylbromid, Acetylbromid, Phosphortribromid oder dem Triphenylphosphin-Brom-Komplex oder Sulfonsäurechloriden wie Methan-, Trifluormethan-, Nonafluorbutan, 2,2,2-Trifluorethan, 4-Methylbenzol-, 4-Brombenzol-, 4-Nitrobenzol oder Benzolsulfonsäurechlorid gegebenenfalls in Gegenwart eines inerten Lösungsmittels und gegebenenfalls in Anwesenheit einer organischen oder anorganischen Base, wobei diese auch gleichzeitig Lösungsmittel sein kann, herstellen (Houben-Weyl-Müller, Methoden der organischen Chemie, Bd. 5/3, Stuttgart 1964, s.760 ff.; Bd. 5/4, Stuttgart 1960, S. 354 ff.; Bd. 9, Stuttgart 1955, S. 388, 663; J. org. Chem. *35*, 3195 (1970)).

Hydroxymethylcycloalkane der Formel II (L = OH) erhält man nach folgendem Schema:

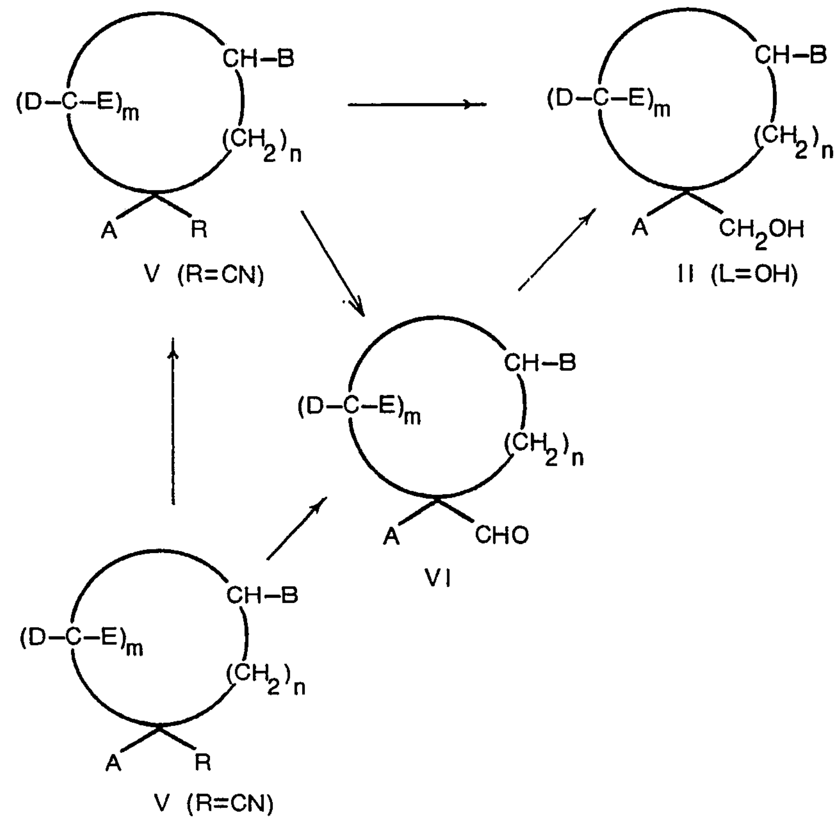

V (R=CN) II (L=OH) VI V (R=CN)

Nitrile der Formel V (R = CN lassen sich sauer oder alkalisch bei Temperaturen zwischen 20 und 120°C in Gegenwart eines Lösungsmittels zu den entsprechenden Carbonsäurederivaten V (R ≠ CN) solvolysieren, die durch Reduktion mit einem komplexen Hydrid, z.B. Lithiumaluminiumhydrid oder Natriumborhydrid — im Falle eines Anhydrids — oder mit Wasserstoff in Gegenwart eines Katalysators drucklos oder unter Druck mehrstufig (V → VI → II) oder einsufig nach bekannten Methoden in die Alkohole II (L = OH) überführt werden.

Man kann aus dem Nitril V (R= CN) durch Reduktion mit Diisobutylaluminiumhydrid (Synthesis *1975*, 617) den Aldehyd VI herstellen, der mit einem komplexen Hydrid, z.B. Natriumborhydrid oder Lithiumaluminiumhydrid oder mit Wasserstoff in Gegenwart eines Katalysators zum Alkohol II (L = OH) weiterreduziert wird.

Die Cycloalkylcarbonsäurederivate V, ikn denen D und E Wasserstoff bedeuten, werden nach üblichen Methoden hergestellt durch Bisalkylierung eines Carbonsäurederivates VII mit einer bifunktionellen Verbindung VIII, in der L eine nucleophil substituierbare Abgangsgruppe ist.

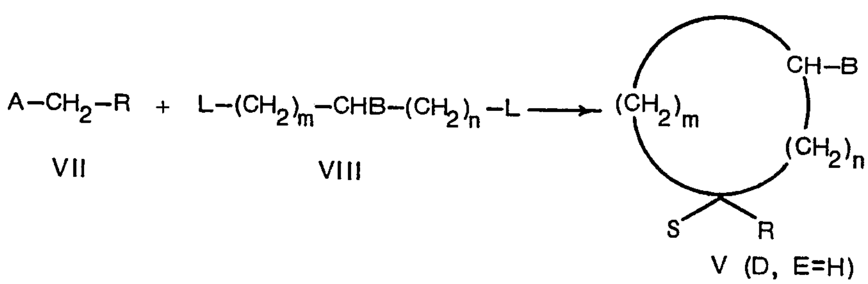

VII VIII V (D, E=H)

Diese Reaktion wird bevorzugt unter Phasen-Transfer-Bedingungen durchgeführt (Rocz. Chem. *40*, 1647 (1966), Przen. Chem. *46*, 393 (1967)), ist jedoch auch in Gegenwart einer starken Base, z.B. eines Metallhydrids oder -amids, möglich (J. Am. Chem. Soc. *69*, 2902 (1947)). Cyclopropylderivate der Formel X, in der A und B die oben angegebene Bedeutung haben, R eine Nitril-, Ester- oder gegebenfalls acetalisierte Aldehydgruppe sein kann und D und E Wasserstoff sind, lassen sich außerdem bevorzugt durch Cyclopropanierung α,β-ungesättigter Nitrile oder Aldehyd- oder Carbonsäurederivate der Formel IX erhalen, in der A, B und R die oben angegebene Bedeutung haben.

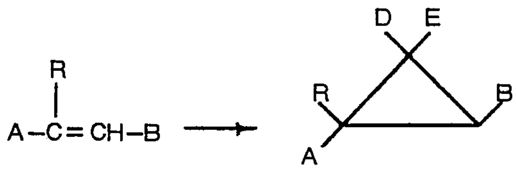

IX X

Für die Reaktion eignen sich besonders Trimethylsulfoxoniumsalze, die in einem inerten Lösungsmittel in Gegenwart einer starken Base, z.B. eines Alkalialkoholates wie Kalium-tert.-butanolat, mit dem α,β-ungesättigten Carbonsäurederivat IX umgesetzt werden. Geht man dabei von stereochemisch einheitlichen Olefinen IX aus, so erhält man die Cyclopropane diastereomerenrein (Z. Naturforsch. *18*, [b], 976 (1976); Chem. Ber. *98*, 3721 (1965)). Weitere Verfahren zur Herstellung von Cyclopropanen durch intramolekulare Reaktion oder durch Umsetzung von Olefinen mit Methylengruppenübertragungsreagentien wie Diazomethan oder nach Simmons-Smith sind beschrieben (Houben-Weyl-Müller, Methoden der organischen Chemie, Stuttgart 1971, Bd. 4/3, S. 32 bis 148).

Cyclopropylderivate der Formel X, in der A und B die oben angegebene Bedeutung haben und R eine Nitril-, Ester- oder eine gegebenenfalls acetalisierte Aldehydgruppe bedeutet und D und E unabhängig voneinander Wasserstoff oder Halogen — mit Ausnahme D und E gleich Wasserstoff — bedeuten, erhält man durch Umsetzung der α,β-ungesättigten Aldehyd- oder Carbonsäurederivate IX mit Mono- oder Dihalogencarbenen, die in der Regel nach klassischen Verfahren (vgl. z. B. Houben-Weyl-Müller, Methoden der organischen Chemie, Stuttgart 1971, Bd. 4/3, S. 150 ff.) oder durch eine Phasen-Transfer-katalysierte Reaktion (Liebigs Ann. *758*, 148 (1972); Synth. Commun. *3* 305 (1973); Tetrahedron *338*, 363 (1977)) in situ erzeugt werden.

Überraschenderweise zeigen die erfindungsgemäßen Azolderivate neben einer guten antibakteriellen und antimykotischen in-vitro-Wirksamkeit eine bessere, therapeutisch nutzbare in-vivo-Wirksamkeit, insbesondere gegen Dermatophyten und Candida, als bekannte Präparate. Die erfindungsgemäßen Wirkstoffe stellen somit eine wertvolle Bereicherung der Pharmazie dar.

Die Wirkung gegen Dermatophyten, Baterien und Protozoen kann nach Methoden, wie sie beispielsweise in P. Klein, Bakteriologische Grundlagen der chemotherapeutischen Laboratoriumspraxis, Springer-Verlag Berlin, 1957, beschrieben wird, aufgezeigt werden. Die überraschende Wirkung gegenüber Hefen wurde im Pseudomyzel- und Myzelphasentest mit Candida albicans nachgewiesen (DE—OS 30 10 093). Es wurde geprüft, welche minimalen Hemmkonzentrationen (MHK-Werte) im Agardilutionstest erreicht werden.

Diese liegen für eine Vielzahl der genannten Beispiele gegen Dermatophyten zwischen 0,5 und 16 µg/ml, gegen parasitäre Hefen im Pseudomyzel- und Myzelphasentest zwischen 0,0078 und 1 µg/ml. Selbst gegen eine Anzahl von Schimmelpilzen liegen die MHK-Werte zwischen 0,25 und 16 µg/ml.

Bei Bakterien wurden Hemmwerte zwischen 1 und 64 µ/ml festgestellt.

Therapierversuche am Tier ergaben, daß erfindungsgemäße Azolderivate nach oraler Gabe gegen Systeminfektionen mit Pilzen und Hefen oder lokale Infektionen, z.B. die Candida Vaginitis der Ratte, hochwirksam und der Referenzsubstanz Ketoconazol überlegen waren. Bei lokaler Behandlung der Candida-Vaginitis der Ratte wurden zur vollen Ausheilung geringe Dosen der hier beschriebenen Azolderivate benötigt. Mit Standardantimykotika, wie z.B. Clotrimazol, Miconazol u.a. waren bei gleicher Behandlungsdauer stets größere Substanzmengen notwendig.

Trichophyten-Infektionen am Meerschweinchen konnten mit geringen Dosen bei lokaler Behandlung ausgeheilt werden.

Die Verbindungen können allein oder zusammen mit anderen bekannten Wirkstoffen, insbesondere Antibiotika, angewendet werden.

Die Herstellung der chemotherapeutischen Mittel oder Zubereitungen mit üblichen festen, halbfesten oder flüssigen Trägerstoffen oder Verdünnungsmitteln und den üblicherweise verwendeten pharmazeutisch-technischen Hilfsstoffen entsprechend der gewünschten Applikationsart mit einer zur Anwendung geeigneten Dosierung erfolgt in üblicher Weise, insbesondere durch Vermischen (vgl. H. Sucker et al., Pharmazeutische Technologie, Thieme-Verlag, Stuttgart, 1978).

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions etc. in Betracht.

Die therapeutisch wirksame Verbindung ist in pharmazeutischen Zubereitungen vorzugsweise in einer Konzentration von 0,5 bis 90 Gew.% der Gesamtmischung vorhanden.

Im allgemeinen können bei oraler Verabfolgung sowohl in der Human- als auch in der Veterinärmedizin der oder die Wirkstoffe in Mengen von etwa 1,0 bis etwa 10,0, vorzugsweise 2 bis 6 mg/kg Körpergewicht pro Tag, vorzugsweise in Form mehrerer Einzelgaben zur Erzielung der gewünschen Ergebnisse verabreicht werden. Es kann jedoch erforderlich sein, von den genannten Dosierungen abzuweichen, und zwar in Abhängigkeit von der Art und der Schwere der Erkrankung, der Art der

Zubereitung und der Applikation des Arzneimittels, sowie dem Zeitraum bzw. Intervall, innerhalb welchem die Verabreichung erfolgt. So kann es in einigen Fällen ausreichend sein, mit weniger als der oben genannten Menge Wirkstoffe auszukommen, während in anderen Fällen die oben angeführte Wirkstoffmenge überschritten werden muß.

Die neuen Verbindungen wirken nicht nur gegen humanpathogene Pilze, sondern können auch als Fungizide in der Landwirtschaft eingesetzt werden.

Sie zeichnen sich ebenso wie ihre Salze und Metallkomplexverbundungen zeichnen sich durch eine hervorraagende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder irhen Samen, insbesondere Weizen, Roggen, Gerste, Hafer, Reis, Mais, Baumwole, Soja, Kaffe, Zuckerrohr, Obst und Zierpflanzen im Gartenbau, sowie Gemüse — wie Gurken, Bohnen und Kürbisgewächse —.

Die neuen Verbindungen sind insbesondere geeignet zur Bekämpfung folgender Pflanzenkrankheiten:
Erysiphe graminis (echter Mehltau) in Getreide,
Ersiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weisen,
Botrytis cinerea (Grauschimmel) an Erdbeeren, Reben,
Cercospora musae an Bananen
Pseudocercosporella herpotrichoides an Weisen, Gerste
Pyricularia oryzae an Reis
Hemileia vastatrix an Kaffee.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion de Pflanzen oder Samen durch die Pilze.

Die neuen Substanzen können in die üblichen Formulierungen übergeführt werden, wie Lösungen, Emulsionen, Suspensionen, Stäube, Pulvre, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Taikum, Kreide und synthetische Gesteinsmehle (z.B. hochdisperse Kieselsäure, Silikate); Emulgiermittel, wie nichtionogene und anionische Emulgatoren (z.B. Polyoxyethylen — Fettalkohol — Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Erffektes zwischen 0,1 und 3 kg Wirkstoff oder mehr je ha.

Die neuen Verbindungen können auch im Materialschutz eingesetzt werden. So lassen sich mit den erfindungsgemäßen Mitteln beispielsweise folgende holz- und austrichverfärbende Pilze, Moderfäulepilze und holzzerstörende Pilze bekämpfen: Pullularia (Aureobasidium) pullulans, Sclerophoma pityophila, Ceratocystis spec., Paecilomyces variotii, Hormiscium spec., Stemphylium spec., Phoma violacea, Cladosporium herbarum, Trichoderma viride, Chaetomium globosum, humicola grisea, Merulius lacrimans, Coniophora puteana, Lentinus lepideus, Lenzites trabea, Polystictus versicolor, Stereum hirsutum, Fomes annosus.

Die neuen Wirkstoffe können im Materialschutz in Zubereitungen, wie Lösunen, Emulsionen, Pasten und Öldispersionen, angewendet werden. Die Zubereitungen enthalten im allgemeinen zwischen 0,1 und 95 Gew.% Wirkstoff, vorzugsweise 0,25 bis 50%. Die Aufwandmengen betragen je nach Art des gewünschten Effektes 0,5 bis 8 g Wirkstoff je $m^2$ zu schützender Holzoberfläche bzw. 50 bis 4000 g Wirkstoff/$m^3$ Holz. Anstrichfarben enthalten 0,5 bis 5 Gew.% Wirkstoff. Zum Schutz von Holzwerkstoffen werden die Wirkstoffe als Emulsion oder im Untermischverfahren dem Klebstoff in Mengen von 2 bis 6 Gew.% zugesetzt. Die Anwendung der Wirkstoffe erfolgt durch Streichen, Spritzen, Sprühen, Tauchen oder Druckimprägnierungs- oder Diffusionsverfahren. Zur Herstellung wasserabweisender Imprägnieranstriche

können den öligen Holzschutzmitteln sog. "water repellents" zugesetzt werden. Geeignete Substanzen sind beispielsweise Zinkstearat, Aluminiumstearat, Wachse. Ferner können zur Erzielung von Farbeffekten anorganische oder organische Pigmente in die Formulierungen eingearbeitet werden.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die neuen Mittel können auch in Mischung mit anderen Wirkstoffen vorliegen, z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des Wirkungsspektrums. Bei einer Anzahl solcher Fungizidmischungen treten auch synergistische Effekte auf, d.h. die Wirksamkeit des Kombinationsproduktes ist größer als die addierten Wirksamkeiten der Einzelkomponenten.

Für die folgenden Versuche wurden bekannte fungizide Wirkstoffe zum Vergleich verwendet, nämlich als Vergleichsmittel A 1-(2,4-Dichlorphenyl)-2-(imidazolyl)-ethan-1-ol, bekannt aus der französischen Patentschrift 2 249 616, als Vergleichsmittel B (2,4-Dichlorphenyl-)-1,2,4-triazol-1-yl-methylketon und als Vergleichsmittel C tert.-Butyl-1,2,4-triazol-1-yl-methylketon, bekannt aus der DE—OS 24 31 407.

## Versuch 1

Wirksamkeit gegen Weizenmehltau

Blätter von in Töpfen gewachsenen Weizenkeimlingen der Sorte "Jubilar" werden mti wäßriger Spritzbrühe, die 80% Wirkstoff und 20% Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 7 Tagen wird das Ausmaß der Mehltauentwicklung ermittelt.

Das Ergbenis des Versuches zeigt, daß die neuen Wirkstoffe 3, 4, 6 bis 10, 16, 18 und 24 z.B. bei der Anwendung als 0,006 oder 0,0015%ige Suspension eine bessere fungizide Wirkung (z.B. 90% zeigten als die bekannten Wirkstoffe A und C (z.B. 79%).

## Versuch 2

Wirksamkeit gegen Weizenbraunrost

Blätter von in Töpfen gewachsenen Weizensämlingen der Sorte "Jubilar" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit wäßrigen Spritzbrühen, die 80% Wirkstoff und 20% Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmaß der Rostpilzentwicklung auf den Blättern ermittelt.

Das Ergebnis des Versuches zeigt, daß die neuen Wirkstoffe 1, 3, 4, 6 bis 10, 16 bis 18, 25 und 90 z.B. bei der Anwendung als 0,025 oder 0,06 %ige Suspension eine bessere fungizide Wirkung (z.B. 97%) zeigten als die bekannten Wirkstoffe A, B und C (z.B. 70%).

## Versuch 3

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80% Wirkstoff und 20% Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß die neuen Wirkstoffe 3, 7, 8, 10, 15, 17 bis 19, 21, 29, 35 und 90 z.B. bei der Anwendung als 0,01 %ige Suspension eine bessere fungizide Wirkung (z.B. 97%) zeigtem als die bekannten Wirkstoffe A, B und C (z.B. 70%).

## Versuch 4

Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus (Erysiphe cichoracearum) besprüht. Nach etwa 20 Stunden werden die Versuchspflanzen mit wäßriger Spritzbrühe, die 80% Wirkstoff und 20% Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22°C und 70 bis 80% relativer Luftfeuchtigkeit aufgestellt. Zur Beurteilung der Wirksamkeit der neuen Stoffe wird das Ausmaß der Pilzentwicklung nach 21 Tagen ermittelt.

Das Ergebnis des Versuches zeigt, daß die neuen Wirkstoffe 6 bis 10, 13, 15 bis 21, 24 bis 28 und 90 z.B. bei der Anwendung als 0,025 %ige Suspension eine sehr gute fungizide Wirkung (z.B. 97%) hatten.

Versuch 5

Wirksamkeit gegen Moderfäule- und Schimmelpilze

Die Wirkstoffe werden — in Aceton gelöst — in Mengen von 40 ppm einem verflüssigten 50 %igen Malzextraktagar zugesetzt. Der Agar wird in Petrischalen ausgegossen und nach dem Erstarren werden die fungizidhaltigen Nähragarplatten zentral mit dem Moderfäule und Stockflecken verursachenden Pilz Chaetomium globosum bzw. dem grünen Holzschimmel Trichoderma viride beimpft. Nach fünftägiger Bebrütung der Schalen bei 25°C wird die Entwicklung der Pilzkolonien auf dem Nährboden im Vergleich mit der Kontrolle (ohne Wirkstoff-Zusatz) beurteilt:

Das Ergebnis des Versuches zeigt, daß die neuen Wirkstoffe 7 bis 10 z.B. bei der Anwendung von 40 ppm Wirkstoff im Nähragar eine sehr gute fungizide Wirkung (z.B. 97%) hatten.

Versuch 6

Fungizide Wirksamkeit gegenüber holzzerstörenden Pilzen

Zur Ermittlung der fungiziden Wirksamkeit gegenüber den holzzerstörenden Pilzen Coniophora puteana und Polystictus versicolor wurden Kiefernsplintholzklötzchen mit den Abmessungen 50 × 25 × 15 mm mit jeweils 100 $g/m^2$ Holzoberfläche öliger Holzschutzmittelzubereitungen, die 1% Wirkstoff enthielten, bestrichen. Nach vierwöchiger Lagerung wurden die behandelten Klötzchen zusammen mit den unbehandelten in Glasschalen gelegt, die als Prüfpilz Coniophora puteana bzw. Polystictus versicolor auf einem Nähragar enthielten. Die Schalen wurden anschließend in einem Klimaraum bei einer Temperatur von 22°C und einer relativen Lufteuchte von 70% bebrütet. Nach dreimonatiger Versuchsdauer wurden die Klötzchen von anhaftendem Pilzmycel befreit und getrocknet. Anschlißend wurde das Ausmaß der Holzzerstörung festgestellt.

Das Ergebnis des Vesuches zeigt, daß die neuen Wirkstoffe 3, 4, 7, 8, 9 und 10 z.B. bei der Anwendung als 1 %ige Zubereitung eine sehr gute fungizide Wirkung (z.B. 100%) zeigten.

Die folgenden Beispiele und Vorschriften erläutern die Herstellung der neuen Verbindungen und ihrer Vorprodukte.

Beispiel 2

a) Herstellung des Ausgangsmaterials

48 g 4-Phenyl-benzaldehyd und 39,9 g 4-Chlor-benzylcyanid wurden mit 3 g Natriummethylat in 400 ml Ethanol 1 h bei 30°C gerührt. Der gebildete Niederschlag wurde abgesaugt, mit wenig Isopropanol und danach mit Petrolether gewaschen. Man erhielt 82,5 g Z-1-(4-Chlorphenyl)-2-(4-biphenylyl)-acrylsäurenitril, Schmp. 194 bis 198°C.

In eine Lösung von 80 g 1-(4-Chlorphenyl)-2-(4-biphenylyl)-acrylsäurenitril und 80 g Trimethylsulfoxoniumiodid in 750 ml Dimethylsulfoxid wurden unter Kühlung bei 15°C und unter Stickstoff 34 g Kalium-tert.butylat portionsweise eingetragen. Man ließ 1 h bei Raumtemperatur nachrühren, goß dann auf Wasser, extrahierte mit Methylenchlorid, wusch die organische Phase mit Wasser, trocknete über Magnesiumsulfat und dampfte das Lösungsmittel im Vakuum ab. Der Rückstand wurde in warmem Ethanol aufgenommen. Daraus kristallisierten 78,6 g 1-(4-Chorphenyl)-2-(4-biphenylyl)-cyclopropylcyanid, Schmp. 129 bis 131°C.

75 g 1-(4-Chlorphenyl)-2-(4-biphenylyl)-cyclopropylcyanid wurden in einem Gemisch aus 500 ml Diethylether und 100 ml Tetrahydrofuran aufgenommen und unter Stickstoff bei 0 bis 4°C tropfenweise mit 300 ml Diisobutylaluminiumhydrid-Lösung (1,2 molar in Toluol) versetzt. Man ließ 1,5 h bei Raumtemperatur rührer, goß dann auf 2 l 10 %ige wäßrige Weinsäurelösung, setzte weitere 2 l Diethylether zu, trente die organische Phase ab, wusch mit Wasser nach und trocknete über Magnesiumsulfat. Beim Einengen der Lösung, bildete sich ein Niederschlag, der abgesaugt und mit Wasser, Ethanol und wenig Petrolether nachgewaschen wurde. Umkristallisieren aus Methanol ergab 73 g 1-(4-Chlorphenyl)-2-(4-biphenylyl)-cyclopropylcarbaldehyd, Schmp. 188 bis 190°C.

73 g 1-(4-Chlorphenyl)-2-(4-biphenylyl)cyclopropylcarbaldehyd wurden in einem Gemisch aus 1 l Methanol sowie 1 l Methylenchlorid 2 h mit 6 g Natriumborhydrid, gerührt. Danach wurde das Lösungsmittel im Vakuum abgedampft, der Rückstand in Methylenchlorid aufgenommen, mit Wasser gewaschen, über Magnesiumsulfat getrocknet und die Lösung zur Trockne eingedampft. Umkristallisieren des Rückstandes aus Toluol erbrachte 68 g 1-(4-Chlorphenyl)-1-hydroxymethyl-2-(4-biphenylyl)-cyclopropan, Schmp. 168 bis 170°C.

b) Herstellung des Endprodukts

Zu 16,5 g 1-(4-Chlorphenyl)-1-hydroxymethyl-2-(4-biphenylyl)-cyclopropan in 150 ml Methylenchlorid und 8,6 ml Triethylamin wurden bei 20°C 4,8 ml Methansulfonsäurechlorid in 15 ml Methylenchlorid getropft. Nach 2 h Rühren bei Raumtemperatur wurde mit verdünnter Natriumhydrogencarbonat-Lösung und anschließend mit Wasser gewaschen, über Magnesiumsulfat getrocknet und das Lösungsmittel im Vakuum abdestilliert.

Der feste Rückstand wurde in 50 ml Dimethylsulfoxid aufgenommen, mit 1 g Kaliumiodid versetzt und

bei Raumtemperatur unter Stickstoff zu eine Lösung getgroft, die aus 4,1 g Imidazol mit 2,4 g Natriumhydrid (80 %ige Dispersion in Mineralöl) in 150 ml Dimethylformamid bei 15°C bereitet worden war. Man ließ 24 h bei Raumtemperatur rühren, trug die Reaktionslösung in Wasser ein, extrahierte mit Methylenchlorid, wusch die organische Phase mit Wasser und trocknete sie über Magnesiumsulfat. Nach Abdampfen des Lösungsmittels blieb ein Sirup zurück, der in warmem Aceton aufgenommen und mit einer gesättigten Lösung von Chlorwasserstoff in Diisopropylether versetzt wurde. Dabei kristallisierten 14,5 g 1-(4-Chlorphenyl)-1-(imidazol-1-yl-methyl)-2-(4-biphenylyl)-cyclopropan als Monohydrochlorid aus, Schmp. 212 bis 214°C.

Entsprechend Beispiel 1 und 2 können die in den Tabellen 1 und 2 aufteführten Verbindungen hergestellt werden bzw. wurden hergestellt:

Tabelle 1 (D = E = Wasserstoff)

| Beispiel | A | B | m | n | Z | Base/Salz | Fp. [°C] |
|---|---|---|---|---|---|---|---|
| 3 | 4-Cl-$C_6H_4$ | 2,4-$Cl_2$-$C_6H_3$ | 1 | 0 | CH | .HCl | 228-234 |
| 4 | 4-Cl-$C_6H_4$ | 2,4-$Cl_2$-$C_6H_3$ | 1 | 0 | N | .HCl | 207-213 |
| 5 | $C_6H_5$ | 4-Cl-$C_6H_4$ | 1 | 0 | CH | | 56-57,5 |
| 6 | $C_6H_5$ | 4-Cl-$C_6H_4$ | 1 | 0 | N | | 94- 96 |
| 7 | 4-Cl-$C_6H_4$ | $C_6H_5$ | 1 | 0 | CH | .HCl | 221-228 |
| 8 | 4-Cl-$C_6H_4$ | $C_6H_5$ | 1 | 0 | N | .HCl | 163-168 |
| 9 | 4-Cl-$C_6H_4$ | 4-Cl-$C_6H_4$ | 1 | 0 | CH | | 86- 88 |
| 10 | 4-Cl-$C_6H_4$ | 4-Cl-$C_6H_4$ | 1 | 0 | N | | 86- 88 |
| 11 | 3,4-$(OCH_3)_2$-$C_6H_3$ | 2,4-$Cl_2$-$C_6H_3$ | 1 | 0 | N | | 117 |
| 12 | 3,4-$(OCH_3)_2$-$C_6H_3$ | 2,4-$Cl_2$-$C_6H_3$ | 1 | 0 | CH | | 121 |
| 13 | 3,4-$(OCH_3)_2$-$C_6H_3$ | 4-Cl-$C_6H_4$ | 1 | 0 | N | | 118-120 |
| 14 | 3,4-$(OCH_3)_2$-$C_6H_3$ | 4-Cl-$C_6H_4$ | 1 | 0 | CH | | 92-116 |
| 15 | 4-Cl-$C_6H_4$ | 3-$CF_3$-$C_6H_4$ | 1 | 0 | CH | | 219-226 |
| 16 | 4-Cl-$C_6H_4$ | 3-$CF_3$-$C_6H_4$ | 1 | 0 | N | .HCl | 126-128 |
| 17 | 4-Br-$C_6H_4$ | 4-$CF_3$-$C_6H_4$ | 1 | 0 | CH | .HCl | 159-162 |
| 18 | 4-Br-$C_6H_4$ | 4-F-$C_6H_4$ | 1 | 0 | N | .HCl | 195-200 |
| 19 | 4-Cl-$C_6H_4$ | 3-(O-$C_6H_5$)-$C_6H_4$ | 1 | 0 | CH | .HCl | 220-225 |
| 20 | 4-Cl-$C_6H_4$ | 3-(O-$C_6H_5$)-$C_6H_4$ | 1 | 0 | N | .HCl | 168-171 |
| 21 | 1-$C_{10}H_7$ | 4-Cl-$C_6H_4$ | 1 | 0 | CH | .$(CO_2H)_2$ | 185-188 |
| 22 | 1-$C_{10}H_7$ | 4-Cl-$C_6H_4$ | 1 | 0 | CH | .HCl | 168-170 |
| 23 | $C_6H_5$ | H | 1 | 0 | N | | 31- 34 |
| 24 | 4-Cl-$C_6H_4$ | H | 1 | 0 | N | .HCl | 145-153 |
| 25 | 4-Cl-$C_6H_4$ | 4-$C_6H_5$-$C_6H_4$ | 1 | 0 | N | .HCl | 174-180 |
| 26 | 4-Cl-$C_6H_4$ | 4-$C(CH_3)_2$-$C_6H_4$ | 1 | 0 | N | .HCl | 135-140 |

| Beispiel | A | B | m | n | Z | Base/Salz | Fp. [°C] |
|---|---|---|---|---|---|---|---|
| 27 | $4-CH_3-C_6H_4$ | $4-Cl-C_6H_4$ | 1 | 0 | N | .HCl | 160-175 |
| 28 | $4-Cl-C_6H_4$ | H | 1 | 0 | CH | .HCl | 199-204 |
| 29 | $4-CH_3-C_6H_4$ | $4-Cl-C_6H_4$ | 1 | 0 | CH | | 84- 86 |
| 30 | $4-Cl-C_6H_4$ | $4-C(CH_3)_3-C_6H_4$ | 1 | 0 | CH | | 98- 99 |
| 31 | $4-Cl-C_6H_4$ | $3,4-Cl_2-C_6H_3$ | 1 | 0 | CH | .HCl | 210-215 |
| 32 | $4-Cl-C_6H_4$ | $3,4-Cl_2-C_6H_3$ | 1 | 0 | N | .HCl | 145-154 |
| 33 | $4-Cl-C_6H_4$ | $2-Cl-C_6H_4$ | 1 | 0 | CH | .HCl | 258-269 |
| 34 | $C_6H_5$ | $2-Cl-C_6H_4$ | 1 | 0 | CH | .HCl | 246-256 |
| 35 | $C_6H_5$ | $4-Cl-C_6H_4$ | 1 | 0 | N | .HCl | 183-189 |
| 36 | $4-Cl-C_6H_4$ | $2-Cl-C_6H_4$ | 1 | 0 | N | .HCl | 177-180 |
| 56 | 3-Furanyl | $4-Cl-C_6H_4$ | 1 | 0 | N | | |
| 57 | 3-Furanyl | $4-Cl-C_6H_4$ | 1 | 0 | CH | | |
| 58 | 3-Thienyl | $4-Cl-C_6H_4$ | 1 | 0 | N | | Harz Öl |
| 59 | 3-Thienyl | $4-Cl-C_6H_4$ | 1 | 0 | CH | | Öl |
| 59a | 3-Thienyl | $4-Cl-C_6H_4$ | 1 | 0 | CH | 1/2 $CuCl_2$ | 121 (Zers.) |
| 60 | 3-Pyridyl | $4-Cl-C_6H_4$ | 1 | 0 | N | | |
| 61 | 3-Pyridyl | $4-Cl-C_6H_4$ | 1 | 0 | CH | | |

| Beispiel | A | B | m | n | Z | Base/Salz | Fp. [°C] |
|---|---|---|---|---|---|---|---|
| 62 | 4-Br-$C_6H_4$ | 4-Cl-$C_6H_4$ | 1 | 0 | N | .HCl | 205-208 |
| 63 | 4-Br-$C_6H_4$ | 4-Cl-$C_6H_4$ | 1 | 0 | CH | | Harz |
| 64 | 4-Cl-$C_6H_4$ | 4-F-$C_6H_4$ | 1 | 0 | N | | 84- 92 |
| 65 | 4-Cl-$C_6H_4$ | 4-F-$C_6H_4$ | 1 | 0 | CH | | Harz |
| 66 | 4-Br-$C_6H_4$ | $C_6H_5$ | 1 | 0 | N | .HCl | 164-170 |
| 67 | 4-Br-$C_6H_4$ | $C_6H_5$ | 1 | 0 | CH | | |
| 68 | 4-Cl-$C_6H_4$ | $CF_3$ | 1 | 0 | CH | | |
| 69 | 4-Cl-$C_6H_4$ | $CF_3$ | 1 | 0 | N | | |
| 70 | 4-Cl-$C_6H_4$ | $CCl_3$ | 1 | 0 | CH | | |
| 71 | 4-Cl-$C_6H_4$ | $CCl_3$ | 1 | 0 | N | | |
| 72 | 4-Cl-$C_6H_4$ | 4-(S-$C_6H_5$)-$C_6H_4$ | 1 | 0 | CH | | |
| 73 | 4-Cl-$C_6H_4$ | 4-(S-$C_6H_5$)-$C_6H_4$ | 1 | 0 | N | | |
| 74 | 4-(S-$C_6H_5$)-$C_6H_4$ | 4-Cl-$C_6H_4$ | 1 | 0 | N | | |
| 75 | 4-(S-$C_6H_5$)-$C_6H_4$ | 2,4-$Cl_2$-$C_6H_3$ | 1 | 0 | N | | |
| 76 | 2,4-$Cl_2$-$C_6H_3$ | 2-Furanyl | 1 | 0 | CH | .HCl | 229-239 |
| 77 | 2,4-Cl-$C_6H_3$ | 2-Furanyl | 1 | 0 | N | .HCl | 155-165 |
| 78 | 4-Cl-$C_6H_4$ | 3-Cl-$C_6H_4$ | 1 | 0 | CH | .HCl | 210-220 |
| 79 | 4-Cl-$C_6H_4$ | 3-Cl-$C_6H_4$ | 1 | 0 | N | .HCl | 152-158 |
| 80 | $C_6H_5$ | 2,6-$Cl_2$-$C_6H_3$ | 1 | 0 | CH | .HCl | 292-294 |
| 81 | $C_6H_5$ | 2,6-$Cl_2$-$C_6H_3$ | 1 | 0 | N | .HCl | 180-182 |

| Beispiel | A | B | m | n | Z | Base/Salz | Fp. [°C] |
|---|---|---|---|---|---|---|---|
| 82 | 4-Cl-$C_6H_4$ | $C(CH_3)_3$ | 1 | 0 | CH | . HCl | 246-250 |
| 83 | 4-Cl-$C_6H_4$ | $C(CH_3)_3$ | 1 | 0 | N | . HCl | 188-192 |
| 84 | 4-Cl-$C_6H_4$ | $CH_3$ | 1 | 0 | CH | | |
| 85 | 4-Cl-$C_6H_4$ | $CH_3$ | 1 | 0 | N | | |
| 86 | 4-Cl-$C_6H_4$ | 2-Thienyl | 1 | 0 | N | . HCl | 165-185 |
| 87 | 4-Cl-$C_6H_4$ | 3-Thienyl | 1 | 0 | N | | |
| 88 | 4-Cl-$C_6H_4$ | 2-Thienyl | 1 | 0 | CH | . HCl | 223-226 |
| 89 | 4-Cl-$C_6H_4$ | 3-Thienyl | 1 | 0 | CH | | |
| 90 | 4-Cl-$C_6H_4$ | 2-Furanyl | 1 | 0 | CH | . HCl | 211-215 |
| 91 | 4-Cl-$C_6H_4$ | 2-Furanyl | 1 | 0 | N | . HCl | 135-145 |
| 92 | 4-Cl-$C_6H_4$ | 4-$C_6H_5$-$C_6H_4$ | 1 | 0 | CH | . HCl | 212-214 |

TABELLE 2

| Beispiel | A | B | D | E | m | n | Z | Base/Salz | Fp. [°C] |
|---|---|---|---|---|---|---|---|---|---|
| 93 | H | $C_6H_5$ | Cl | Cl | 1 | 0 | N | . HCl | 140-144 |
| 94 | H | $4-Cl-C_6H_4$ | Cl | Cl | 1 | 0 | N | . HCl | 161-168 |
| 95 | $4-Cl-C_6H_4$ | $C_6H_5$ | Cl | Cl | 1 | 0 | CH | | |
| 96 | $4-Cl-C_6H_4$ | $C_6H_5$ | Cl | Cl | 1 | 0 | N | | |
| 97 | H | $4-Cl-C_6H_4$ | F | F | 1 | 0 | N | | |
| 98 | H | $4-Cl-C_6H_4$ | F | F | 1 | 0 | CH | | |
| 99 | $4-F-C_6H_4$ | $2,4-Cl_2-C_6H_4$ | H | H | 1 | 0 | CH | . HCl | 194-197 |
| 100 | $4-F-C_6H_4$ | $2,4-Cl_2-C_6H_4$ | H | H | 1 | 0 | N | . HCl | 170-179 |
| 101 | $C_6H_5$ | $4-Cl-C_6H_4$ | F | F | 1 | 0 | N | | |
| 102 | $C_6H_5$ | H | F | F | 1 | 0 | CH | | |
| 103 | $C_6H_5$ | H | F | F | 1 | 0 | N | | |
| 104 | $4-Cl-C_6H_4$ | H | F | F | 1 | 0 | CH | | |
| 105 | $4-Cl-C_6H_4$ | H | F | F | 1 | 0 | N | | |
| 106 | $4-Cl-C_6H_4$ | H | H | Br | 1 | 0 | CH | | |
| 107 | $4-Cl-C_6H_4$ | H | H | Br | 1 | 0 | N | | |
| 108 | $4-Cl-C_6H_4$ | H | Cl | Cl | 1 | 0 | CH | | |
| 109 | $4-Cl-C_6H_4$ | H | Cl | Cl | 1 | 0 | N | | |
| 110 | $C_6H_5$ | H | Cl | Cl | 1 | 0 | CH | | |
| 111 | $C_6H_5$ | H | Cl | Cl | 1 | o | N | | |

Galenische Zubereitungen

Beispiel A

Tablette mit 250 mg Wirkstoff

Zusammensetzung für 1000 Tabletten:

| | |
|---|---|
| Wirkstoff des Beispiels Nr. 3 | 250 g |
| Kartoffelstärke | 100 g |
| Milchzucker | 50 g |
| Gelatinelösung 4% | 45 g |
| Talkum | 10 g |

Herstellung:

Der fein gepulverte Wirkstoff, Kartoffelstärke und Milchzucker werden gemischt. Die Mischung wird mit ca. 45 g 4 %iger Gelatinelösung durchfeuchtet, feinkörnig granuliert und getrocknet. Das trockene Granulat wird gesiebt, mit 10 g Talkum vermischt und auf einer Rundläufer-Tablettiermaschine zu Tabletten verpreßt. Die Tabletten werden in dicht schließende Behälter aus Polypropylen gefüllt.

Beispiel B

Creme mit 1% Wirkstoff

| | |
|---|---|
| Wirkstoff des Beispiels Nr. 3 | 1,0 g |
| Glycerinmonostearat | 10,0 g |
| Cetylalkohol | 4,0 g |
| Polyethylenglykol-400-stearat | 10,0 g |
| Polyethylenglykol-sorbitanmonostearat | 10,0 g |
| Propylenglykol | 6,0 g |
| p-Hydroxybenzoesäuremethylester | 0,2 g |
| Entmineralisiertes Wasser | ad 100,0 g |

Herstellung:

Der feinst gepulverte Wirkstoff wird mit Propylenglykol suspendiert und die Suspension in die auf 65°C erwärmte Schmelze aus Glycerinmonostearat, Cetylalkohol, Polyethylenglykol-400-stearat und Polyethylenglykolsorbitanmonostearat gerührt. In diese Mischung wird die 70°C heiße Lösung des p-Hydroxybenzoesäuremethylester in Wasser emulgiert. Nach dem Erkalten wird die Creme in einer Kolloidmühle homogenisiert und in Tuben abgefüllt.

Beispiel C

Puder mit 1% Wirkstoff

| | |
|---|---|
| Wirkstoff des Beispiels Nr. 3 | 1,0 g |
| Zinkoxid | 10,0 g |
| Magnesiumoxid | 10,0 g |
| Hochdisperses Siliciumdioxid | 2,5 g |
| Magnesiumstearat | 1,0 g |
| Talkum | 75,5 g |

Herstellung

Der Wirkstoff wird in einer Luftstrahlmühle mikronisiert und mit den anderen Bestandteilen homogen vermischt. Die Mischung wird durch ein Sieb (Maschenweite Nr. 7) geschlagen und in Polyethylenbehälter mit Streueinsatz abgefüllt.

Beispiel I

20 Gewichtsteile der Verbindung des Beispiels 2 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammemühle vermahlen. Durch feines Verteilen der Mischung in Wasser erhält man eine Spritzbrühe.

Beispiel III

30 Gewichtsteile der Verbindung des Beispiels 4 werden mit einer Mischung aus 92 Gewichtsteilen pulverförmigem Kieselsäuregel und 8 Gewichtsteilen Paraffinöl, das auf die Obefläche dieses Kieselsäuregels gesprüht wurde, innig vermischt. Man erhält auf diese Weise eine Aufbereitung des Wirkstoffs mit guter Haftfähigkeit.

Beispiel IV

40 Gewichtsteile der Verbindung des Beispiels 7 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit Wasser erhält man eine wäßrige Dispersion.

Beispiel V

20 Teile der Verbindung des Beispiel 9 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Beispiel VI

Man vermischt 90 Gewichtsteile der Verbindung des Beispiels 17 mit 10 Gewichtsteilen N-Methylalpha-pyrrolidon und erhält eine Lösung, die zur Anwendung in Form kleinster Tropfen geeignet ist.

Beispiel VII

20 Gewichtsteile der Verbindung des Beispiels 4 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausießen und feines Verteilen der Lösung in Wasser erhält man eine wäßrige Dispersion.

Beispiel VIII

20 Gewichtsteile der Verbindung des Beispiels 9 werden in einer Mischung gelöst, die aus 40 Gewichtsteillen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

Beispiel IX

10 Gewichtsteile der Verbindung des Beispiels 12, 20 Gewichtsteile Polyoxyethylensorbitanmololaurat, 20 Gewichtsteile Methanol und 50 Gew.-Teile Wasser werden zu einer Lösung verrührt, die 10 Gew.% des Wirkstoffs enthält. Durch Hinzufügen von weiterem Wasser können stärker verdünnte Lösungen hergestellt werden.

Beispiel X

Zur Herstellung eines lösemittelhaltigen Holzschutzmittels mit 1% Wirkstoff wird zunächst 1 Teil (Gewichtsteil) der Verbindung des Beispiels 3 unter leichtem Erwärmen in 55 Teilen einer aromatenreichen Benzinfraktion gelöst. Anschließend werden 10 Teile eines Alkydharzes zugefügt und bei Raumtemperatur mit Testbenzin auf 100 teile ergänzt.

Analog Beispiel I bis X können lösemittelhaltige Holzschutzmittel mit bis zu 5 Gew.% Wirkstoffgehalt hergestellt werden.

**Patentansprüche**

1. Azolylmethylcycloalkane der Formel I

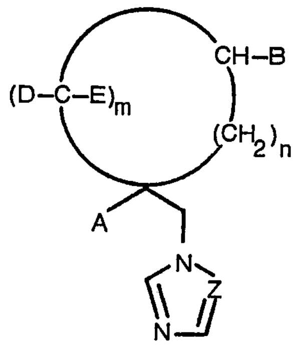

(I),

in der

A und B gleich oder verschieden sind und Wasserstoff, Alkyl mit 1 bis 4 C-Atomen, die gegebenenfalls durch Halogen substituiert sein können, Naphthyl, 2- bzw. 3-Furanyl, 2- bzw. 3-Thienyl, 2-, 3-, oder 4-Pyridyl, Biphenyl oder Phenyl bedeuten, wobei der Phenylrest durch Halogen, Nitro, Alkyl, Alkoxy oder Halogenalkyl mit jeweils 1 bis 4 C-Atomen, Phenoxy oder den Phenylsulfenylrest substituiert sein kann,

D und E gleich oder verschieden sind und Wasserstoff oder Halogen bedeuten,

Z CH oder N bedeutet,

m 1 und

n 0 bedeuten,

sowie deren verträgliche Säureadditionssalze und Metallkomplexe.

2. Azolylmethylcyclopropane der Formel I gemäß Anspruch 1, dadurch gekennzeichnet, daß D und E Wasserstoff sind.

3. Verfahren zur Herstellung von Azolylmethylcycloalkane der Formel I

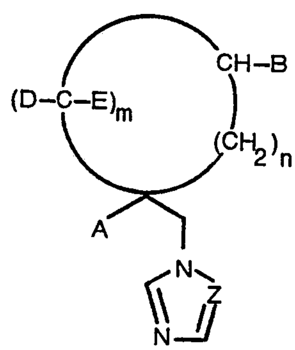

(I),

in der

A und B gleich oder verschieden sind und Wasserstoff, Alkyl mit 1 bis 4 C-Atomen, die gegebenenfalls durch Halogen substituiert sein können, Naphthyl, 2- bzw. 3-Furanyl, 2- bzw. 3-Thienyl, 2-, 3-, oder 4-Pyridyl, Biphenyl oder Phenyl bedeuten, wobei der Phenylrest durch Halogen, Nitro, Alkyl, Alkoxy oder Halogenalkyl mit jeweils 1 bis 4 C-Atomen, Phenoxy oder den Phenylsulfenylrest substituiert sein kann,

D und E gleich oder verschieden sind und Wasserstoff oder Halogen bedeuten,

Z CH oder N bedeutet,

m 1 und

n 0 bedeuten,

sowie deren verträgliche Säureadditionssalze und Metallkomplexe, dadurch gekennzeichnet, daß man

a) ein Cycloalkan der Formel II

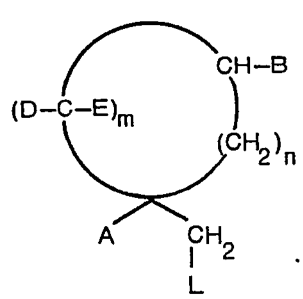

(II),

in der A, B, D, E, m und n die oben angegebene Bedeutung haben und L eine nucleophil substituierbare Abgangsgruppe darstellt, mit einer Verbindung der Formel III

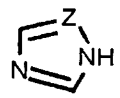

(III),

in der Z die oben angegebene Bedeutung hat, umsetzt oder

b) eine Verbindung der Formel II, in der A, B, D, m und n die oben angegebene Bedeutung haben und L für eine Hydroxygruppe steht, mit einer Verbindung der Formel IV,

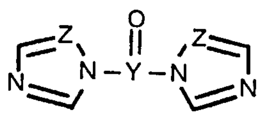

(IV)

in welcher Z die oben angegebene Bedeutung hat, und Y eine Kohlenstoff- oder Schwefelatom bedeutet, in einem aprotischen Lösungsmittel umsetzt
und die so erhaltenen Verbindungen gegebenenfalls in ihre verträglichen Säureadditionssalze oder Metallkomplexe überführt.

4. Verfahren gemäß Anspruch 3, dadurch gekennzeichnet, daß man Verbindungen der Formel I gemäß Anspruch 3 herstellt, in denen D und E Wasserstoff sind.

5. Arzneimittel, enthaltend ein Azolylmethylcycloalkan gemäß Anspruch 1.

6. Fungizides Mittel, enthaltend ein Azolylmethylcycloalkan gemäß Anspruch 1.

7. Fungizides Mittel, enthaltend ein Azolylmethylcycloalkan gemäß Anspruch 1 und inerte Zusatzstoffe.

8. Verfahren zur Bekämpfung von Pilzen, dadurch gekennzeichnet, daß man ein Azolylmethylcycloalkan der Formel I gemäß Anspruch 1 auf die Pilze oder auf durch Pilzbefall bedrohte Materialien, Flächen, Pflanzen oder Saatgüter einwirken läßt.

**Revendications**

1. Azolylméthylcycloalcanes de la formule I

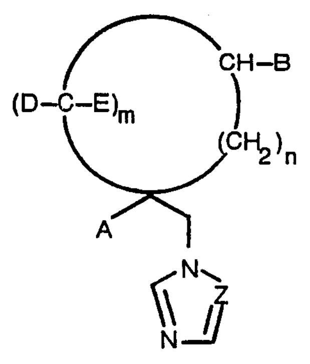

(I),

dans laquelle

A et B sont identiques ou différents et représentent hydrogène, alkyle de 1 à 4 atomes C qui peuvent éventuellement être substitués par halogène, naphtyle, 2- ou 3- furanyle, 2- ou 3- thiényle, 2-, 3- ou 4-pyridyle, biphényle ou phényle, le reste phényle pouvant être substitue par halogène, nitro, alkyle, alcoxy ou halogénoalkyle ayant chacun 1 à 4 atomes C, phénoxy ou le reste phénylsulfényle,

D et E sont identiques ou différents et représentent hydrogène ou halogène

Z représente CH ou N

m représente 1 et

n représente 0

ainsi que leurs sels d'addition d'acide et complexes métalliques compatibles.

2. Azolylméthylcyclopropanes de la formule I selon la revendication 1, caractérisé par le fait que D et E sont l'hydrogène.

3. Procédé de préparation d'azolylméthylcyclalcanes de la formule I

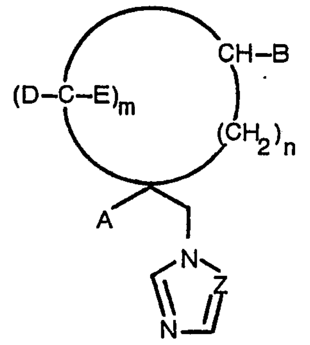

(I),

dans laquelle

A et B sont identiques ou différents et représentent hydrogène, alkyle de 1 à 4 atomes C qui peuvent éventuellement être substitués par halogène, naphtyle, 2- ou 3- furanyle, 2- ou 3- thiényle, 2-, 3- ou 4-pyridyle, biphényle ou phényle, le reste phényle pouvant être substitue par halogène, nitro, alkyle, alcoxy ou halogénoalkyle ayant chacun 1 à 4 atomes C, phénoxy ou le reste phénylsulfényle,

D et E sont identiques ou différents et représentent hydrogène ou halogène

Z représente CH ou N

m représente 1 et

n représente 0

ainsi que leurs sels d'addition d'acide et complexes métalliques compatibles, caractérisé par le fait que:

a) on fait réagir un cycloalcane de la formule II

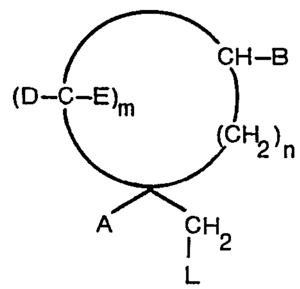

(II),

dans laquelle A, B, D, E, m et n ont les significations sus-indiquée et L représente un groupe éliminable substituable, nucléophyle avec un composé de la formule III

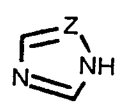

(III),

dans laquelle Z a la signification sus-indiquée

ou

b) on fait réagir dans un solvant aprotique un composé de la formule II dans laquelle A, B, D, E, m et n ont les significations sus-indiquées et L est mis pour un groupe hydroxy, avec un composé de la formule IV

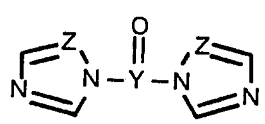

(IV)

dans laquelle Z a la signification sus-indiquée et Y représente un atome de carbone ou de soufre, et on transforme éventuellement les composés ainsi obtenus en leurs sels d'addition d'acide ou complexes métalliques compatibles.

4. Procédé selon la revendication 3, caractérisé par le fait qu'on prépare des composés de la formule I selon la revendication 3 dans laquelle D et E sont l'hydrogène.

5. Médicament, contenant un azolylméthylcycloalcane de formule I selon la revendication 1.

6. Agent fongicide contenant un azolylméthylcycloalcane de formule I selon la revendication 1.

7. Agent fongicide, contenant un azolylméthylcycloalcane de formule I selon la revendication 1, et des additifs inertes.

8. Procédé de lutte contre les champignons, caractérisé par le fait qu'on fait agir un azolylméthylcycloalcane de formule I selon la revendication 1 sur les champignons ou sur les surfaces, plantes ou semences, menacées d'une attaque par champignons.

**Claims**

1. An azolylmethylcycloalkane of the formula I

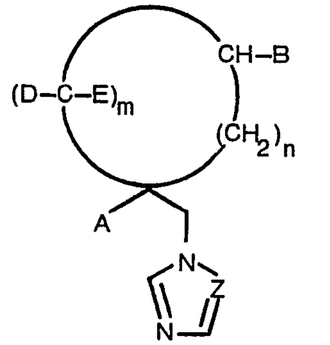

(I),

where

A and B are identical or different and are each hydrogen or alkyl of 1 to 4 carbon atoms which may be substituted by halogen, or naphthyl or 2- or 3-furanyl, 2- or 3-thienyl, 2-, 3- or 4-pyridyl, biphenyl or phenyl, where the phenyl radical can be substituted by halogen, nitro, alkyl, alkoxy or haloalkyl, each of 1 to 4 carbon atoms, or by phenoxy or phenylsulfenyl,

D and E are identical or different and are each hydrogen or halogen,

Z is CH or N,

m is 1 and

n is 0,

and its tolerated acid addition salts and metal complexes.

2. An azolylmethylcyclopropane of the formula I as claimed in claim 1, wherein D and E are each hydrogen.

3. A process for the preparation of an azolylmethylcycloalkane of the formula I

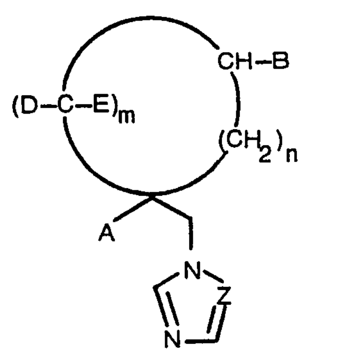

(I),

where

A and B are identical or different and are each hydrogen or alkyl of 1 to 4 carbon atoms which may be substituted by halogen, or naphthyl or 2- or 3-furanyl, 2- or 3-thienyl, 2-, 3- or 4-pyridyl, biphenyl or phenyl, where the phenyl radical can be substituted by halogen, nitro, alkyl, alkoxy or haloalkyl, each of 1 to 4 carbon atoms, or by phenoxy or phenylsulfenyl,

D and E are identical or different and are each hydrogen or halogen,

Z is CH or N,

m is 1 and

n is 0,

and its tolerated acid addition salts and metal complexes, wherein

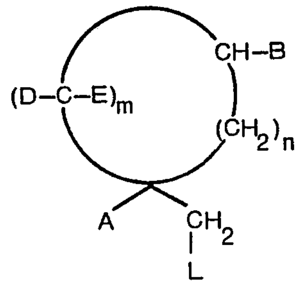

(II),

where A, B, D, E, m and n have the abovementioned meanings and L is a nucleophilically substitutable leaving group, is reacted with a compound of the formula III

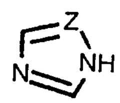

(III),

where Z has the abovementioned meanings, or

b) a compound of the formula II, where A, B, D, E, m and n have the abovementioned meanings and L is a hydroxyl group, is reacted with a compound of the formula IV

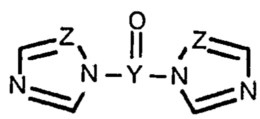

(IV)

where Z has the abovementioned meanings and Y is a carbon or sulfur atom, in an aprotic solvent, and the compound thus obtained is, if required, converted into its tolerated acid addition salts or metal complexes.

4. A process as claimed in claim 3, wherein a compound of the formula I in which D and E are each hydrogen is prepared as claimed in claim 3.

5. A drug containing an azolylmethylcycloalkane as claimed in claim 1.

6. A fungicide containing an azolylmethylcycloalkane as claimed in claim 1.

7. A fungicide containing an azolylmethylcycloalkane as claimed in claim 1 and inert additives.

8. A method of controlling fungi, wherein an azolylmethylcycloalkane of the formula I as claimed in claim 1 is allowed to act on the fungi or on materials, areas, plants or seeds threatened by fungal infestation.